# EUROPÄISCHE PATENTSCHRIFT

(11) **EP 1 337 194 B1**
(45) Veröffentlichungstag und Bekanntmachung des Hinweises auf die Patenterteilung: **03.12.2008**
(21) Anmeldenummer: 01983597.4
(22) Anmeldetag: 21.11.2001
(51) Int. Cl.: A61B 18/12

(54) **VERFAHREN ZUM BETREIBEN EINES INSTRUMENTS FÜR DIE HF-CHIRURGIE UND ELEKTROCHIRURGISCHE VORRICHTUNG**
METHOD FOR OPERATING AN INSTRUMENT FOR USE IN HIGH-FREQUENCY SURGERY, AND AN ELECTROSURGICAL DEVICE
PROCEDE SERVANT A FAIRE FONCTIONNER UN INSTRUMENT DE CHIRURGIE HF ET DISPOSITIF ELECTROCHIRURGICAL

(30) Priorität: 21.11.2000 DE 10057585
(43) Veröffentlichungstag der Anmeldung: 27.08.2003
(73) Patentinhaber: Erbe Elektromedizin GmbH, 72072 Tübingen (DE)
(72) Erfinder: BELLER, Jürgen, 72810 Gomaringen (DE); EISELE, Florian, 72074 Tübingen (DE); FISCHER, Klaus, 72202 Nagold (DE)
(74) Vertreter: Bohnenberger, Johannes
(86) Internationale Anmeldenummer: PCT/EP2001/013537
(87) Internationale Veröffentlichungsnummer: WO 2002/041798

(56) Entgegenhaltungen:
- EP-A1- 0 947 167
- DE-A1- 4 339 049
- US-A- 6 074 386
- US-A- 6 165 173

## Beschreibung

Die Erfindung betrifft ein Verfahren zum Betreiben eines Instruments für die HF-Chirurgie sowie eine elektrochirurgische Vorrichtung.

Elektrochirurgische Geräte, die mit hochfrequenten Strömen betrieben werden, haben in den letzten Jahren zunehmend an Bedeutung gewonnen. Sie umfassen im allgemeinen ein vom Operateur handhabbares Instrument sowie mindestens ein Gerät, an welches dieses angeschlossen ist. Das Gerät liefert zum einen einen hochfrequenten elektrischen Strom, zum anderen werden über das Gerät "Hilfsmittel", wie beispielsweise die Zufuhr eines Edelgases, das Absaugen von beim Operieren entstehendem Rauch, Spüleinrichtungen oder dergleichen Zusatzgeräte gesteuert. Mit derartigen elektrochirurgischen Vorrichtungen kann eine Vielzahl von chirurgischen Eingriffen unter den verschiedensten Bedingungen, sowohl in der offenen Chirurgie als auch in der (minimalinvasiven) endoskopisch betriebenen Chirurgie durchgeführt werden, wobei Gewebe geschnitten, koaguliert, "verklebt" oder in sonstiger Weise bearbeitet wird.

Einerseits haben derartige Geräte den großen Vorteil, daß sie sehr spezifisch auf die durchzuführende Operation auch unter Berücksichtigung der Arbeitsgewohnheiten des Operateurs einstellbar sind, andererseits bringt dies auch den großen Nachteil mit sich, den jeder schon beim Einstellen seines Videorecorders oder Autoradios bemerkt hat: Es gibt einfach "zu viele Einstellmöglichkeiten, man findet sich nicht mehr zurecht."

Aus der DE 43 39 049 Al (D1) ist eine Einrichtung zur Konfiguration chirurgischer Systeme bekannt. Dabei wird ein elektrochirurgisches Instrument mit einer Kodiereinrichtung ausgestattet, über die eine für das Instrument charakteristische Kennung mittels einer Dekodiereinrichtung an einen Mikroprozessor gesendet werden kann. Über den Mikroprozessor lässt sich dann ein HF-Chirurgiegerät, an das das Instrument angeschlossen ist, entsprechend der Kodierung hinsichtlich eines geeigneten Betriebsmodus für das Instrument einstellen.

Der Erfindung liegt die Aufgabe zugrunde, ein Verfahren zum Betreiben eines HF-chirurgischen Instrumentes bzw. eine elektrochirurgische Vorrichtung anzugeben, die in einfacher und anwendungsoptimaler Art und Weise verwendbar sind.

Diese Aufgabe wird durch ein Verfahren nach Anspruch 1 sowie eine Vorrichtung nach Anspruch 7 gelöst.

Ein wesentlicher Punkt der Erfindung liegt darin, daß eine individuell definierte Konfiguration HF-chirurgischer Systeme für ein HF-chirurgisches Instrument ermöglicht wird. Es kann also der Operateur die von ihm aufgefundene und sowohl auf den Operationszweck als auch auf seine persönlichen, individuellen Gewohnheiten, Fähigkeiten und Vorlieben zugeschnittenen Einstellungen durch einfaches Anstecken "seines" Instrumentes an ein vorhandenes Gerät nicht nur leicht wieder auffinden, er kann diese Einstellungen vielmehr sofort übernehmen. Ein Instrumentenwechsel ist somit ohne weiteres möglich, da der Operateur sofort das Instrument mit den Betriebsdaten betreibt, die er kennt und vorzufinden wünscht. Wenn er während einer Operation Veränderungen der Betriebsdaten wünscht, so kann er diese in der gewohnten Art und Weise am Gerät vornehmen und - wenn ihm diese Betriebsart besser erscheint - auch übernehmen. Es findet hier also eine Individualisierung der Instrumente statt, die der Operateur benutzt. Er hat also damit "sein" Operationsbesteck, das er immer mit sich nehmen kann.

Insbesondere wird die o.g. Aufgabe durch ein Verfahren zum Betreiben eines Instruments für die HF-Chirurgie an mindestens einem Gerät für die HF-Chirurgie eines HF-chirurgischen Systems gelöst, welches die nachfolgenden Schritte umfaßt:
- Erfassen von Betriebsdaten des Gerätes mindestens während einer ersten Benutzung des an das Gerät angeschlossenen Instruments, wobei als Betriebsdaten von einem Operateur individuell und/oder werkseitig wählbare Einstelldaten zur Vorgabe einer definierten Konfiguration des HF-chirurgischen Systems für das HF-chirurgische Instrument vorgesehen sind, wobei diese erste Benutzung des Gerätes sowohl an einem Modell als auch während einer wirklichen Operation stattfinden kann;
- Senden der Betriebsdaten an eine zum Speichern der Betriebsdaten ausgebildete Speichereinrichtung und Speichern der Betriebsdaten, die während dieser ersten Benutzung als optimal aufgefunden wurden, wobei die Speichereinrichtung mit dem Instrument verbunden ist, derart, daß sie im Instrument oder in einer Steckereinrichtung zum Verbinden des Instruments mit dem Gerät angebracht ist;
- Senden der Betriebsdaten an eine Datenerfassungseinrichtung bei einer erneuten oder weiteren Benutzung und/oder einer Überprüfung des Instruments, um die zuvor als optimal aufgefundenen Betriebsdaten nun wieder dem Gerät mitzuteilen und dieses Gerät auf eben diese Betriebsdaten einzustellen;
- Einstellen des Gerätes entsprechend den Betriebsdaten während der ersten Benutzung derart, daß das Instrument wie bei der ersten Benutzung betreibbar ist und/oder Auswerten dieser Betriebsdaten zur Überprüfung des Instruments, so daß die definierte Konfiguration des Systems eingestellt wird.

Hierbei ist unter einer "ersten" Benutzung die jeweils vor einer weiteren Benutzung vorausgehende Benutzung zu verstehen, also nicht unbedingt die allererste Benutzung des Instrumentes.

Unter Betriebsdaten ist im Minimalfall "Betrieb", "Pause" zu verstehen, wobei anhand derartiger Betriebsdaten natürlich nur dokumentiert werden kann, wann und wie oft das Gerät benutzt wurde, um dadurch einen verbesserten Service oder auch eine Dokumentation über die Arbeit des Operateurs zur Verfügung zu stellen. Bei einem APC-Gerät, wie es beispielsweise in der WO 97/11647 beschrieben ist, wären unter Betriebsdaten die eingestellte Spannung, die "Stromform" und der eingestellte Zufuhrstrom von Edelgas zu verstehen. Dieser Betriebsdaten, die der Operateur z.B. bei einer sehr problematisch einzustellenden Operation im Oesophagus aufgrund seiner Erfahrung und seiner Übung erarbeitet, stehen ihm bei seiner nächsten Operation ohne weiteres zur Verfügung. Hierbei ist es auch möglich, die Speichereinrichtungen eines Instrumentes, das für eine Vielzahl von verschiedenen Operationen verwendbar ist, derart zu gestalten, daß vom Operateur aufgefundene "Programme", die auf die jeweilige Operation optimiert sind, abgerufen werden können.

Vorzugsweise werden die momentanen Betriebsdaten auf ein insbesondere manuell eingebbares Speicherbefehlssignal hin gespeichert. Der Operateur bestimmt also den Zeitpunkt, zu welchem er die Betriebsdaten eingestellt hat, die er in Zukunft insbesondere für den besonderen, gerade erfüllten Zweck weiterverwenden will.

Zu Service- und insbesondere auch zu Dokumentationszwecken des Operateurs ist es von Vorteil, wenn die Betriebsdaten den Benutzungszeitraum, das Benutzungsdatum od.dgl. überwachungsrelevante Servicedaten umfassen. Dadurch ist es dem Operateur möglich, die von ihm durchgeführten Operationen besonders akurat aufzuzeichnen, so daß ein exakter "Lernvorgang" auf wissenschaftlicher Basis ermöglicht wird. Selbstverständlich können auch derartige Daten zu haftungsrelevanten Zwecken verwendet werden.

Vorzugsweise umfassen die Betriebsdaten auch Benutzeridentifikationsdaten, die vom Benutzer eingebbar sind. Auf diese Weise kann das Instrument noch erheblich besser als durch ein einfaches Namensschild individualisiert werden, wodurch - bei entsprechender Anzeige der Benutzeridentifikationsdaten - eine Verwechslung von Instrumenten sicher vermieden werden kann.

Im Instrument werden weiterhin Identifikationsdaten und zwar vorzugsweise werkseitig und unabänderbar gespeichert, die bei Verbindung mit einem Gerät diesem insbesondere zur Vornahme von Grundeinstellungen von Betriebsdaten übermittelt werden. Diese Grunddaten sind derart ausgewählt, daß sie nicht in Widerspruch zu den vom Operateur ermittelten und gespeicherten Betriebsdaten stehen, diese also nicht "überschreiben". Bei einem "jungfräulichen" Instrument können diese Betriebsdaten die Grundeinstellungen für den allgemeinen Betrieb darstellen, von denen ausgehend der Operateur den "Optimalbetrieb" ermittelt. Sobald dann im Instrument bzw. in der dazugehörigen Speichereinrichtung die vom Operateur nun aufgefundenen optimalen Betriebsdaten gespeichert sind, werden die werkseitig vorgespeicherten Grundbetriebsdaten nicht mehr verwendet. Es ist dann aber dem Operateur wiederum möglich, im Laufe von Versuchen vorgenommene Fehleinstellungen, die "ins Chaos" geführt haben, rückgängig zu machen, indem wieder auf die werkseitigen Grundeinstellungen zurückgegriffen wird.

Vorrichtungsmäßig wird die Aufgabe durch eine elektrochirurgische Vorrichtung gelöst, welche folgende Merkmale umfaßt:
- mindestens ein Gerät für die Elektrochirurgie eines HF-chirurgischen Systems, insbesondere ein HF-Generator;
- ein von einem Operateur handhabbares Instrument für die HF-Elektrochirurgie, das nach Anschließen an einen Patientenstromkreis des Geräts zur Durchführung von Behandlungen biologischen Gewebes verwendbar ist;
- eine Betriebsdatenerfassungseinrichtung zum Erfassen von momentan am Gerät und an gegebenenfalls mit diesem zusammen verwendbaren Zusatzeinrichtungen eingestellten Betriebsdaten, wobei als Betriebsdaten von einem Operateur individuell und/oder werkseitig wählbare Einstelldaten zur Vorgabe einer definierten Konfiguration des HF-chirurgischen Systems für das HF-chirurgische Instrument vorgesehen sind;
- eine zum Speichern der Betriebsdaten ausgebildete Speichereinrichtung, die mit dem Instrument verbunden ist, wobei darauf hingewiesen wird, daß diese Speichereinrichtung im Instrument selbst oder in einer Steckereinrichtung zum Verbinden des Instruments mit dem Gerät vorgesehen sein kann;
- eine bi-direktionale Datenübermittlungseinrichtung, insbesondere ein Datenbus zum Senden der Betriebsdaten vom Gerät zum Instrument und von gespeicherten Daten vom Instrument zum Gerät,
so daß die definierte Konfiguration des Systems einstellbar ist.

Vorzugsweise ist am Gerät eine manuell betätigbare Befehlseinrichtung, z.B. ein Tastenschalter, zum Senden der momentan eingestellten Betriebsdaten in die Speichereinrichtungen vorgesehen, so daß diese Betriebsdaten in der Speichereinrichtung gespeichert werden. Eine derartige Befehlseinrichtung kann auch in einem Handschalter am Instrument oder in einem Fußschalter realisiert sein.

Die Speichereinrichtungen sind - je nach der Größe des Instruments - im Instrument selbst oder in einer Steckereinrichtung, mit welcher das Instrument mit dem Gerät verbunden werden kann, vorgesehen. Wichtig ist hierbei, daß zwischen der Speichereinrichtung und dem Instrument eine fehlerfrei auffindbare bzw. nicht trennbare Verbindung besteht, da ja die Individualisierung des Instruments durch den Inhalt der dazu gehörigen Speichereinrichtung stattfindet.

Das Gerät, also z.B. der HF-Generator, weist eine bi-direktionale Hilfs-Datenübermittlungseinrichtung auf, also z.B. einen Steckeranschluß für einen Datenbus, zum Verbinden mit den Zusatzeinrichtungen, z.B. einer Ventileinrichtung für eine Gasquelle, derart, daß aus dem Instrument stammende Betriebsdaten zur Einstellung der Zusatzeinrichtung und Betriebsdaten der Zusatzeinrichtungen zum Speichern in den Speichereinrichtungen übermittelbar sind. Auf diese Art und Weise können auch sehr komplexe und darum eine nicht unerhebliche Zeit und Erfahrung zur Optimierung der Einstellung benötigende Geräteanordnungen in allereinfachster Weise betrieben werden.

Im Gerät sind vorzugsweise Zeit- und/oder Datumserzeugungseinrichtungen (z.B. eine Uhr) vorgesehen, deren Ausgangsdaten in den Speichereinrichtungen in Übereinstimmung mit Betriebsdaten, insbesondere mit Benutzungszeitpunkten des Instruments, vorzugsweise unter gleichzeitiger Speicherung von dazugehörigen Betriebsparametern gespeichert werden. Mit derartigen Einrichtungen ist eine optimale Dokumentation, wie sie oben geschildert wurde, möglich. Weiterhin ist es möglich, betriebskritische Daten, wie z.B. Benutzungszeitdauer und - intensitäten mit vorgegebenen Werten zu vergleichen und dann ein Warnsignal abzugeben, wenn eine Wartung des Instrumentes oder gar ein Ersetzen des Instrumentes durch ein neues Instrument aus Sicht des Herstellers zur Aufrechterhaltung eines optimalen Betriebs wünschenswert oder gar notwendig sind.

Zu Service- und/oder Dokumentationszwecken ist vorzugsweise eine Ausleseeinrichtung zum Auslesen und/oder Ausdrucken der in den Speichereinrichtungen gespeicherten Daten vorgesehen. Diese Ausleseeinrichtung kann sowohl im Gerät (oder einem damit verbundenen separaten Gerät) oder aber in einer ganz gesonderten Einrichtung vorgesehen sein, die unabhängig von dem HF-chirurgischen Gerät betreibbar ist. In diesem Fall nimmt der Benutzer "seine Speichereinrichtung" zur Benutzung mit einem bestimmten Instrumententyp.

Zum Eingeben von Benutzeridentifikationsdaten in die Speichereinrichtungen, also zur weiteren Individualisierung eines Instruments ist im Gerät oder in einem Zusatzgerät eine Tastatur, eine Schnittstelle (zum Anschließen eines PC) od.dgl. Dateneingabeeinrichtung vorgesehen. Der Benutzer kann also hier seine persönlichen Daten, z.B. seinen Namen sowie gegebenenfalls zusätzlich den besonderen Verwendungszweck eingeben, auf den er das Instrument (bzw. die in ihm gespeicherten Betriebsdaten) optimiert hat. Hier können auch verschiedene Betriebsprogramme, die - wie oben angegeben - zugehörig zu verschiedenen Operationssituationen z.B. unter Kennziffern gespeichert wurden, wiedergegeben werden, wenn ein Instrument für verschiedene Operationssituationen optimiert wurde.

Von Vorteil ist es, wenn ein vom Benutzer unveränderbarer Speicher zum insbesondere werkseitigen Speichern von instrumentenspezifischen Identifikations- und/oder Betriebsdaten des Instruments vorgesehen ist. Dieser Speicher kann entweder ein ROM oder aber ein durch den Benutzer nicht zugänglicher Speicherbereich eines zum Speichern der Betriebsdaten vorgesehenen EEPROMS sein. Durch die in diesem unveränderbaren Speicher vorgesehenen Daten erfährt das Instrument zum einen eine werkseitige Individualisierung (Losnummer), zum anderen können hier Grund-Betriebsdaten gespeichert werden, die bei einer Verwendung des Instrumentes zum allerersten Male das damit verbundene HF-chirurgische Gerät einstellen oder eine Rückkehr zu einer Grundeinstellung ermöglichen.

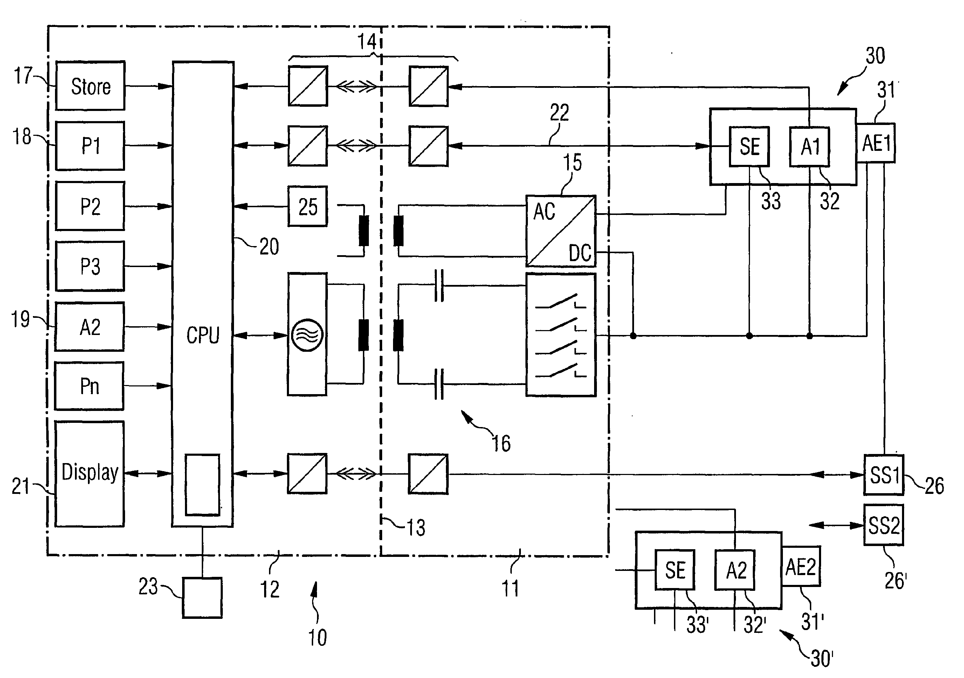

Mit nachfolgenden Ausführungsbeispielen wird die Erfindung anhand der beiliegenden Abbildung erläutert.

In der Abbildung ist - stark schematisiert - ein Gerät 10 gezeigt, das in diesem Fall als HF-Generator ausgebildet ist. Im Gerät 10 ist durch eine Isolationsstrecke 13 ein Patientenstromkreis 11 von einem Zwischenstromkreis 12 getrennt.

Das Gerät 10 weist weiterhin eine Rechen/Steuereinheit 20 auf, die als CPU bezeichnet wird. Die CPU 20 steuert eine HF-Generatorschaltung 16, die durch einen Betätigungsschalter 19, der beispielsweise als Fußschalter ausgebildet ist, in Gang gesetzt wird. Die Betriebsparameter werden vom Operateur am Gerät 10 durch Einstellorgane 18 (Einstellorgane P1-Pₙ vorgewählt. Betriebsdaten und weitere Daten, wie sie weiter unten noch erläutert werden, können auf einem Display 21 zur Anzeige gebracht werden.

Mit dem Gerät 10 kann ein Instrument 30 über eine Steckverbindung verbunden werden. Im vorliegenden Beispiel wird als Instrument 30 ein multifunktionales Instrument beschrieben, das sowohl zum HF-chirurgischen Schneiden als auch zum HF-chirurgischen Koagulieren von Gewebe verwendbar ist. Zum Koagulieren wird aus einer Zusatzeinrichtung 26, im vorliegenden Beispielsfall wäre dies eine Gasversorgung, ein Edelgas im Instrument 30 bzw. einem daran vorgesehenen aktiven Teil 31 zugeführt, wobei die Steuerung der Gasversorgung bzw. der Zusatzeinrichtung 26 wie in der Abbildung gezeigt über die CPU 20 entsprechend den an den Einstellorganen 18 vorgenommenen Einstellungen erfolgt.

Bei einer hier nicht gezeigten Ausführungsform der Erfindung sind mehrere derartige, aber verschieden ausgebildete Instrumente 30 an das Gerät 10 anschließbar.

Im Instrument 30 bzw. mit diesem fest verbunden sind ein Speicher 33 und ein Signalschalter 32 vorgesehen. Der Speicher 33 steht über eine bidirektionale Verbindung 22 ebenso wie der Signalschalter 32 über Optokoppler 14 mit der CPU 20 in Verbindung. Zur Versorgung des Speichers 33 ist im Gerät 10 eine Instrmentenstromversorgung 15 vorgesehen.

Bei der ersten Inbetriebnahme eines Instruments 30, wenn dieses also an das Gerät 10 angesteckt wird, werden in der an sich bekannten Weise über eine im Instrument 30 vorgesehene und werkseitig programmierte Festspeicheranordnung oder über eine Steckercodierung oder dgl. Identifizierungseinrichtung Instrumentendaten in die CPU 20 eingelesen, woraufhin die CPU 20 Grundeinstellungen vornimmt, die für das angesteckte Instrument 30 einen Grundbetrieb ermöglichen. Der Operateur nimmt nun die ihm zutreffend erscheinenden verfeinerten Einstellungen über die Einstellorgane 18 vor, wobei entsprechend der gerade durchgeführten Operation bestimmte Einstelldaten von ihm als optimal aufgefunden werden. Sobald diese "Optimaldaten" vorliegen, wird eine Speichertaste 17 am Gerät 10 betätigt, woraufhin die CPU 20 die Einstellungen der Einstellorgane 18 ausliest und diese Einstellungen über die bi-direktionale Verbindung 22 dem Speicher 33 im Instrument 30 übermittelt, der diese Einstellungen speichert. Soll in einem darauffolgenden Betrieb, also an einem anderen Ort und/oder zu einem anderen Zeitpunkt eine Operation erfolgen, welche dieselben Betriebsdaten erfordert, so werden beim Anstecken des Instrumentes 30 an das Gerät 10 die gespeicherten Betriebsdaten über die bi-direktionale Verbindung 22 der CPU 20 übermittelt, die daraufhin alle zum Speicherzeitpunkt gewählten Einstellungen der Einstellorgane 18 vornimmt. Darüber hinaus werden auch die von der CPU 20 der Zusatzeinrichtung 26 übermittelten Steuerbefehle beim Speichern der Betriebsparameter, also die optimale Einstellung, mitgespeichert und bei einem nächsten Betrieb wieder zur Einstellung der Zusatzeinrichtung 26 vorgegeben.

Wenn mehrere Instrumente 30, 30' gleichzeitig an einem einzigen Gerät 10 verwendet werden, so wird über eine Betätigung des Signalschalters 32 der CPU 20 mitgeteilt, welches der angesteckten Instrumente gerade Verwendung findet, so daß die CPU 20 die in dem jeweiligen Speicher 33 bzw. 33' gespeicherten Einstelldaten übernimmt und die Betriebsparameter (auch die der Zusatzeinrichtung 26) entsprechend einstellt.

Weiterhin ist eine Tastatur 23 vorgesehen, über welche eine Individualisierung der Instrumente 30, 30' derart vorgenommen werden kann, daß z.B. der das Instrument 30, 30' verwendende Operateur seinen Namen oder eine besondere Bezeichnung des optimierten Verwendungszwecks über die Tastatur 23 eingibt und durch Betätigen der Speichertaste 17 über die bi-direktionale Verbindung 22 in den Speicher 33, 33' einliest. Bei einem erneuten Anstecken eines Gerätes 30, 30' an ein entsprechend ausgebildetes Gerät 10 werden dann auf dem Display 21 der Name des Operateurs und der Verwendungszweck des Instruments 30, 30' angezeigt, so daß der Operateur genau weiß, welches seiner eigenen Instrumente gerade angesteckt wurde.

Bei einer hier nicht gezeigten Ausführungsform der Erfindung ist entweder der Signalschalter 32 entsprechend ausgebildet oder ein zusätzlicher Signalschalter vorgesehen, über welchen der CPU 20 mitgeteilt wird, welches von mehreren im Speicher 33, 33' gespeicherten "Einstellprogramme" nunmehr Verwendung finden soll. Dies ist insbesondere dann von großem Vorteil, wenn ein Gerät während einer Operation für verschiedene Zwecke, also in verschiedenen optimalen Parameter-Konfigurierungen Verwendung findet. Derartiges kann z.B. bei der Anwendung verschiedener Koagulationsarten, die jeweils vom Operateur optimiert wurden, vorteilhaft sein.

Aus obigem geht hervor, daß ein Grundgedanke der Erfindung in der Individualisierung der Instrumente 30 liegt, die im Zusammenhang mit einem Gerät 10 Verwendung finden und zwar derart, daß der Operateur seine, als optimal aufgefundenen Betriebsweisen dem Gerät 10 und daran angeschlossenen Zusatzeinrichtungen 26 mitteilt, ohne diese Einstellungen von Hand wieder vornehmen zu müssen.

Weiterhin ist im Gerät 10 ein Zeitbaustein 25 vorgesehen, über welchen Zeit- und Datumsignale der CPU 20 mitgeteilt werden, welche diese Signale in Übereinstimmung mit Betriebsarten über die bi-direktionale Verbindung 22 im Speicher 33 speichert. Dadurch ist eine Dokumentation der "Betriebsvergangenheit" eines jeden Instrumentes 30, 30' möglich, so daß zum einen durchgeführte Operationen optimal dokumentiert und zum anderen dem Servicepersonal alle Daten an die Hand gegeben werden können, die eine zuverlässige und dennoch nicht übertriebene Wartung ermöglichen. Hierzu ist es auch möglich, anstelle einer Anzeige dieser Daten über das im Gerät 10 vorgesehene Display 21 in einem gesonderten Gerät vornehmen zu lassen, welches zwar die bi-direktionale Verbindung 22 und auch eine Stromversorgung für den Speicher 33, jedoch nicht den HF-Stromkreis und gegebenenfalls andere Verbindungen aufweist.

### Bezugszeichenliste

- 10: Gerät
- 11: Patientenstromkreis
- 12: Zwischenstromkreis
- 13: Isolationsstrecke
- 14: Optokoppler
- 15: Instrumentenversorgung
- 16: HF-Generatorschaltung
- 17: Speichertaste
- 18: Einstellorgan
- 19: Fußschalter
- 20: CPU
- 21: Display
- 22: Bi-direktionale Verbindung
- 23: Tastatur
- 25: Zeitbaustein
- 26: Zusatzeinrichtung
- 30: Instrument
- 31: aktives Teil
- 32: Signalschalter
- 33: Speicher

## Patentansprüche

1. Verfahren zum Betreiben eines Instruments für die HF-Chirurgie an mindestens einem Gerät für die HF-Chirurgie eines HF-chirurgischen Systems, umfassend die Schritte
- Erfassen von Betriebsdaten des Gerätes (10) mindestens während einer ersten Benutzung des an das Gerät (10) angeschlossenen Instruments (30), wobei als Betriebsdaten insbesondere über Einstellorgane (18) und eine Speichertaste (17) von einem Operateur individuell und/oder werkseitig wählbare Einstelldaten zur Vorgabe einer definierten Konfiguration des HF-chirurgischen Systems für das HF-chirurgische Instrument vorgesehen sind;
- Senden der Betriebsdaten an eine zum Speichern der Betriebsdaten ausgebildete Speichereinrichtung (33) und Speichern der Betriebsdaten,
wobei die Speichereinrichtung (33) mit dem Instrument (30) verbunden ist, derart, daß sie im Instrument (30) oder in einer Steckereinrichtung zum Verbinden des Instruments (30) mit dem Gerät (10) angebracht ist;
- Senden der Betriebsdaten an eine Datenerfassungseinrichtung (20) bei einer erneuten oder weiteren Benutzung und/oder einer Überprüfung des Instruments (30);
- Einstellen des Gerätes (10) entsprechend den Betriebsdaten während der ersten Benutzung derart, daß das Instrument (30) wie bei der ersten Benutzung betreibbar ist und/oder Auswerten dieser Betriebsdaten zur Überprüfung des Instruments (30),
so daß die definierte Konfiguration des Systems eingestellt wird.

2. Verfahren nach Anspruch 1,
**dadurch gekennzeichnet, daß**
die momentanen Betriebsdaten auf ein insbesondere manuell eingebbares Speicherbefehlsignal hin gespeichert werden.

3. Verfahren nach einem der vorhergehenden Ansprüche,
**dadurch gekennzeichnet, daß**
die momentanen Betriebsdaten auch solche umfassen, die einer Fluidquelle, insbesondere Edelgasquelle, einer Absaugeinrichtung oder dgl. Zusatzeinrichtung zugeordnet sind.

4. Verfahren nach einem der vorhergehenden Ansprüche,
**dadurch gekennzeichnet, daß**
die Betriebsdaten Benutzungszeitraum-, Benutzungsdatum- oder dgl. überwachungsrelevante Servicedaten umfassen.

5. Verfahren nach einem der vorhergehenden Ansprüche,
**dadurch gekennzeichnet, daß**
die Betriebsdaten Benutzeridentifikationsdaten umfassen, die vom Benutzer eingebbar sind.

6. Verfahren nach einem der vorhergehenden Ansprüche,
**dadurch gekennzeichnet, daß**
im Instrument Identifikationsdaten, vorzugsweise werkseitig und vorzugsweise unabänderbar gespeichert und bei Verbindung mit einem Gerät diesem insbesondere zur Vornahme von Grundeinstellungen von Betriebsdaten übermittelt werden.

7. Elektrochirurgische Vorrichtung, umfassend
- mindestens ein Gerät (10) für die HF-Elektrochirurgie eines HF-chirurgischen Systems;
- mindestens ein von einem Operateur handhabbares Instrument (30) für die HF-Elektrochirurgie, das nach Anschließen an einen Patientenstromkreis (11) des Gerätes (10) zur Durchführung von Behandlungen biologischen Gewebes verwendbar ist;
- eine Betriebsdatenerfassungseinrichtung (20) zum Erfassen von momentan am Gerät (10) und an gegebenenfalls mit diesem zusammen verwendeten Zusatzeinrichtungen (26, 26') eingestellten Betriebsdaten,
wobei als Betriebsdaten insbesondere über Einstellorgane (18) und eine Speichertaste (17) von einem Operateur individuell und/oder werkseitig wählbare Einstelldaten zur Vorgabe einer definierten Konfiguration des HF-chirurgischen Systems für das HF-chirurgische Instrument vorgesehen sind;
- eine zum Speichern der Betriebsdaten ausgebildete Speichereinrichtung (33), die mit dem Instrument (30) verbunden ist,
derart, daß sie im Instrument (30) oder in einer Steckereinrichtung zum Verbinden des Instruments (30) mit dem Gerät (10) angebracht ist;
- eine bi-direktionale Datenvermittlungseinrichtung (22, 14) zum Senden der Betriebsdaten vom Gerät (10) zum Instrument (30) und von gespeicherten Daten vom Instrument (30) zum Gerät (10),
so daß die definierte Konfiguration des Systems einstellbar ist.

8. Elektrochirurgische Vorrichtung nach Anspruch 7,
**gekennzeichnet durch** eine vorzugsweise manuell betätigbare Befehlseinrichtung (17) zum Senden der momentan eingestellten Betriebsdaten an die Speichereinrichtung (33) derart, daß diese in der Speichereinrichtung (33) gespeichert werden.

9. Elektrochirurgische Vorrichtung nach einem der Ansprüche 7 oder 8,
**dadurch gekennzeichnet, daß**
das Gerät (10) bi-direktionale Hilfs-Datenübermittlungseinrichtungen aufweist zum Verbinden der Zusatzeinrichtungen (26) derart, daß aus dem Instrument (30) stammende Betriebsdaten zur Einstellung der Zusatzeinrichtung (26) und Betriebsdaten der Zusatzeinrichtung (26) zum Speichern in den Speichereinrichtungen (33) übermittelbar sind.

10. Elektrochirurgische Vorrichtung nach einem der Ansprüche 7 bis 9,
**gekennzeichnet durch** Zeit- und /oder Datumserzeugungseinrichtungen (25), deren Ausgangsdaten in den Speichereinrichtungen (33), insbesondere mit Benutzungszeitpunkten des Instruments (30) vorzugsweise unter gleichzeitiger Speicherung von dazugehörigen Betriebsparametern gespeichert werden.

11. Elektrochirurgische Vorrichtung nach Anspruch 10,
**gekennzeichnet durch** eine Ausleseeinrichtung (20/23) zum Auslesen und/oder Ausdrucken in den Speichereinrichtungen (33) gespeicherter Daten zu Service- und/oder Dokumentationszwecken.

12. Elektrochirurgische Vorrichtung nach einem der Ansprüche 7 bis 11,
**gekennzeichnet durch** eine Tastatur (23), eine Schnittstelle oder dgl. Dateneingabeeinrichtung zum Eingeben von Benutzeridentifikationsdaten in die Speichereinrichtungen (33).

13. Elektrochirurgische Vorrichtung nach einem der Ansprüche 7 bis 12,
**gekennzeichnet durch** einen vom Benutzer unveränderbaren Speicher zum vorzugsweise werkseitigen Speichern von instrumentenspezifischen Identifikations- und/oder Betriebsdaten des Instruments (30).

## Claims

1. Method of operating an instrument for use in HF surgery with at least one device for HF surgery in an HF surgery system, comprising the steps of
- acquiring operational data for the device (10), at least during initial use of the instrument (30) connected to the device (10),
wherein set-up data, which can be selected as operational data individually by an operator, in particular by means of setting mechanisms (18) and a memory key (17), and/or as default, are provided to specify a defined configuration of the HF surgery system for the HF surgical instrument;
- transmitting the operational data to a storage means (33) configured to store the operational data, and storing the operational data,
wherein the storage means (33) is connected to the instrument (30) in such a manner that it is accommodated in the instrument (30) or in a connector mechanism for connecting the instrument (30) to the device (10);
- transmitting the operational data to a data acquisition device (20) during resumed or further use and/or when testing the instrument (30);
- setting the device (10) according to the operational data during the initial use such that the instrument (30) can be operated in the same manner as during the initial use, and/or evaluating this operational data to test the instrument (30) so that the defined configuration of the system is set.

2. Method according to Claim 1,
**characterised in that**
the current operational data are stored in response to a storage-command signal, which is capable of being entered manually in particular.

3. Method according to one of the previous claims,
**characterised in that**
the current operational data also comprise such data that are assigned to a fluid source, in particular a noble gas source, of a suction mechanism or similar auxiliary device.

4. Method according to one of the previous claims,
**characterised in that**
the operational data comprise data relating to the period of use, date of use or similar service data relevant to maintenance.

5. Method according to one of the previous claims,
**characterised in that**
the operational data comprise user identification data, which can be entered by the user.

6. Method according to one of the previous claims,
**characterised in that**
identification data are stored in the instrument, preferably as default and preferably in an unalterable manner, and, when the instrument is connected to the device, are transmitted to the latter in particular to effect basic settings of operational data.

7. Electrosurgical apparatus, comprising
- at least one device (10) for HF electrosurgery in an HF surgery system;
- at least one instrument (30) for use in HF electrosurgery, which can be handled by an operator and, once connected to a patient circuit (11) of the device (10), can be used to perform treatment of biological tissue;
- an operational data acquisition unit (20) for recording operational data currently set on the device (10) and, where applicable, on auxiliary devices (26, 26') used together with the latter,
wherein set-up data, which can be selected as operational data individually by an operator, in particular by means of setting mechanisms (18) and a memory key (17), and/or as default, are provided to specify a defined configuration of the HF surgery system for the HF surgical instrument;
- a storage means (33) configured to store the operational data, which is connected to the instrument (30) in such a manner that it is accommodated in the instrument (30) or in a connector mechanism for connecting the instrument (30) to the device (10);
- a bidirectional data switching device (22, 14) for transmitting the operational data from the device (10) to the instrument (30) and stored data from the instrument (30) to the device (10) so that the defined configuration can be set.

8. Electrosurgical apparatus according to Claim 7,
**characterised by** a command device (17), which can be preferably manually actuated, for sending the currently set operational data to the storage means (33) in such a manner that they are stored in the storage means (33).

9. Electrosurgical apparatus according to one of the Claims 7 or 8,
**characterised in that**
the device (10) has bidirectional ancillary data switching means for connecting the auxiliary devices (26) in such a manner that operational data originating from the instrument (30) can be transmitted to set an auxiliary device (26) and operational data from the auxiliary device (26) can be transmitted for storing in the storage means (33).

10. Electrosurgical apparatus according to one of the Claims 7 to 9,
**characterised by** time and/or date generation means (25), the output data from which are stored in the storage means (33), in particular with times of use of the instrument (30) preferably by simultaneous storage of associated operating parameters.

11. Electrosurgical apparatus according to Claim 10,
**characterised by** a selection device (20/23) for selecting and/or printing out data stored in the storage means (33) for service and/or documentation purposes.

12. Electrosurgical apparatus according to one of the Claims 7 to 11,
**characterised by** a keyboard (23), an interface or similar data entry device for entering user identification data in the storage means (33).

13. Electrosurgical apparatus according to one of the Claims 7 to 12,
**characterised by** a memory that cannot be altered by the user for preferably default storage of instrument-specific identification data and/or operational data of the instrument (30).

## Revendications

1. Procédé permettant de faire fonctionner un instrument de chirurgie HF sur au moins un appareil de chirurgie HF d'un système de chirurgie HF, comprenant les étapes consistant à :
- saisir des données opérationnelles de l'appareil (10) au moins pendant une première utilisation de l'instrument (30) raccordé à l'appareil (10), des données de réglage sélectionnables individuellement par un opérateur et/ou en usine, notamment au moyen d'organes de réglage (18) et d'une touche de mémorisation (17), étant prévues en tant que données opérationnelles pour paramétrer une configuration définie du système de chirurgie HF pour l'instrument de chirurgie HF ;
- envoyer les données opérationnelles à un dispositif de stockage (33) conçu pour mémoriser les données opérationnelles et mémoriser lesdites données opérationnelles, le dispositif de stockage (33) étant relié à l'instrument (30) de telle façon qu'il est disposé dans l'instrument (30) ou dans un dispositif de connexion servant à relier l'instrument (30) à l'appareil (10) ;
- envoyer les données opérationnelles à un dispositif d'acquisition de données (20) lors d'une nouvelle ou d'une autre utilisation et/ou lors d'un contrôle de l'instrument (30) ;
- régler l'appareil (10) en fonction des données opérationnelles pendant la première utilisation de telle façon que l'instrument (30) puisse fonctionner comme lors de la première utilisation et/ou exploiter ces données opérationnelles pour vérifier l'instrument (30) afin de régler la configuration définie du système.

2. Procédé selon la revendication 1, **caractérisé en ce que** les données opérationnelles à un moment donné sont mémorisées lors de l'envoi d'un signal de commande de mémorisation qui peut notamment être saisi manuellement.

3. Procédé selon l'une des revendications précédentes, **caractérisé en ce que** les données opérationnelles à un moment donné comprennent également des données associées à une source de fluide, en particulier une source de gaz rare, à un dispositif d'aspiration ou à un dispositif additionnel similaire.

4. Procédé selon l'une des revendications précédentes, **caractérisé en ce que** les données opérationnelles comprennent des données de maintenance concernant la période d'utilisation, la date d'utilisation ou des données de surveillance similaires.

5. Procédé selon l'une des revendications précédentes, **caractérisé en ce que** les données opérationnelles comprennent des données d'identification d'utilisateur qui peuvent être saisies par l'utilisateur.

6. Procédé selon l'une des revendications précédentes, **caractérisé en ce que** des données d'identification sont mémorisées dans l'instrument, de préférence en usine et de préférence de manière non modifiable, et, lors de la connexion à un appareil, transmises à ce dernier, en particulier pour effectuer des réglages de base de données opérationnelles.

7. Dispositif électrochirurgical, comprenant
- au moins un appareil (10) pour l'électrochirurgie HF d'un système de chirurgie HF ;
- au moins un instrument (30) manipulable par un opérateur pour l'électrochirurgie HF et utilisable après raccordement de l'appareil (10) à un circuit électrique patient (11) pour effectuer des traitements sur des tissus biologiques ;
- un dispositif d'acquisition de données opérationnelles (20) servant à acquérir des données opérationnelles paramétrées à un moment donné au niveau de l'appareil (10) et au niveau de dispositifs additionnels (26, 26') le cas échéant utilisés avec celui-ci, des données de réglage sélectionnables individuellement par un opérateur et/ou en usine, notamment au moyen d'organes de réglage (18) et d'une touche de mémorisation (17), étant prévues en tant que données opérationnelles pour paramétrer une configuration définie du système de chirurgie HF pour l'instrument de chirurgie HF ;
- un dispositif de stockage (33), conçu pour mémoriser les données opérationnelles, qui est relié à l'instrument (30) de telle façon qu'il est disposé dans l'instrument (30) ou dans un dispositif de connexion servant à relier l'instrument (30) à l'appareil (10) ;
- un dispositif de transmission de données bidirectionnelle (22, 14) servant à envoyer les données opérationnelles de l'appareil (10) à l'instrument (30) et des données mémorisées de l'instrument (30) à l'appareil (10) de façon à pouvoir régler la configuration définie du système.

8. Dispositif électrochirurgical selon la revendication 7, **caractérisé par** un dispositif de commande (17), de préférence actionnable manuellement, servant à envoyer les données opérationnelles paramétrées à un moment donné au dispositif de stockage (33) de telle façon que ces données sont mémorisées dans le dispositif de stockage (33).

9. Dispositif électrochirurgical selon la revendication 7 ou la revendication 8, **caractérisé en ce que** l'appareil (10) présente des dispositifs auxiliaires de transmission de données bidirectionnelle servant à relier les dispositifs additionnels (26) de telle façon que les données opérationnelles de réglage du dispositif additionnel (26) provenant de l'instrument (30) et les données opérationnelles du dispositif additionnel (26) puissent être transmises en vue de leur mémorisation dans les dispositifs de stockage (33).

10. Dispositif électrochirurgical selon l'une des revendications 7 à 9, **caractérisé par** des dispositifs générateurs de temps et/ou de date (25) dont les données de sortie sont mémorisées dans les dispositifs de stockage (33), en particulier avec les moments d'utilisation de l'instrument (30), de préférence en mémorisant simultanément des paramètres de fonctionnement correspondants.

11. Dispositif électrochirurgical selon la revendication 10, **caractérisé par** un dispositif de lecture (20/23) servant à lire et/ou imprimer les données mémorisées dans les dispositifs de stockage (33) à des fins de maintenance et/ou de documentation.

12. Dispositif électrochirurgical selon l'une des revendications 7 à 11, **caractérisé par** un clavier (23), une interface ou un dispositif de saisie de données similaire servant à saisir des données d'identification d'utilisateurs dans les dispositifs de stockage (33).

13. Dispositif électrochirurgical selon l'une des revendications 7 à 12, **caractérisé par** une mémoire non modifiable par l'utilisateur servant à mémoriser de préférence en usine des données d'identification spécifiques à l'instrument et/ou des données opérationnelles de l'instrument (30).
